# EUROPEAN PATENT SPECIFICATION

(11) **EP 4 351 686 B1**
(45) Date of publication and mention of the grant of the patent: **25.06.2025**
(21) Application number: 21732272.6
(22) Date of filing: 10.06.2021
(51) Int. Cl.: A61M 16/00, A61M 16/12, A61M 16/20, A61M 16/10, A61M 16/04, A61M 16/06

(54) **DEVICE AND SYSTEM FOR PPV AND CPAP TREATMENT**
VORRICHTUNG UND SYSTEM ZUR PPV- UND CPAP-BEHANDLUNG
DISPOSITIF ET SYSTÈME DE TRAITEMENT PAR PPV ET CPAP

(43) Date of publication of application: 17.04.2024
(73) Proprietor: Neores AB, 831 45 Östersund (SE)
(72) Inventor: DREVHAMMAR, Thomas, 831 43 Östersund (SE); NILSSON, Kjell, 831 45 Östersund (SE)
(74) Representative: AWA Sweden AB
(86) International application number: PCT/EP2021/065646
(87) International publication number: WO 2022/258185

(56) References cited:
- EP-A1- 1 064 043
- WO-A2-2011/060204
- US-A1- 2006 000 475
- US-A1- 2013 327 332

## Description

### FIELD OF THE INVENTION

The present invention relates to the field of devices and systems for positive pressure ventilation (PPV) and continuous positive airway pressure (CPAP) treatment, and to a method of adjusting expiratory time constants and imposed work of breathing in such treatments.

### BACKGROUND OF THE INVENTION

Devices and a systems for positive pressure ventilation (PPV) and continuous positive airway pressure (CPAP) treatment are known in the art for neonatal resuscitation and initial respiratory support. Some known devices and systems have been designed and tested for preterm and term neonatal use.

Traditionally, there are two different systems for positive pressure ventilation in use, i.e. the simple T-piece or a bag. Both systems use a mask interface to the child but an endotracheal (ET) tube may also be used. The two systems are easy to handle, uncomplicated, inexpensive, and have been in use for many decades.

The T-piece systems are able to provide positive pressure ventilation as well as CPAP for a breathing infant but the bag systems are unable to provide CPAP for the breathing infant.

In neonatal resuscitation there is a need for both ventilation (PPV) of the non breathing child and support by CPAP for the spontaneously breathing child. The need for both types of support is common and changes over time during the resuscitation period.

Both the T-piece and the bag systems allow easy positive pressure ventilation for the non-breathing child but only the T-piece systems can also provide CPAP. However, these systems expose the neonate to a higher imposed work of breathing than most of the specialised CPAP systems, which may lead to an extended period of PPV. This could be overcome by switching between two systems; one for PPV and a specialised system for CPAP. Switching between the two systems is, however, unpractical during the resuscitation period.

There was a need to achieve an easy switch between PPV and CPAP for respiratory support without change of equipment and to provide a low imposed work of breathing for the breathing child treated with CPAP. These problems were solved by a device and system as descried in WO2012108826, which combines easy provision of both PPV and CPAP compatible with nasal interfaces and face masks, as well as with endotracheal tubes.

The device and system disclosed in WO2012108826 is pressure stable and provides low resistance to breathing for the treated patient. This low resistance to breathing has been suggested and assumed to be beneficial. There are, however, currently no systems that allow investigation of the effects of the level of resistance to breathing and, thus, that allow optimizing the resistance to breathing provided by such devices and systems. Resistance to breathing is directly related to expiratory time constants during PPV and to imposed work of breathing during CPAP. Adjustments of expiratory time constants and work of breathing for individual patients, research subjects or animals is not possible with technology and systems available today.

### SUMMARY OF THE INVENTION

It is an object to mitigate, alleviate, or eliminate one or more of the above-identified deficiencies in the art and disadvantages singly or in any combination, and to provide a solution to the above-mentioned problem.

To better address this concern, in a first aspect of the invention there is presented a device for positive pressure ventilation (PPV) and continuous positive airway pressure (CPAP) treatment comprising a fresh gas flow inlet arranged to receive a fresh gas flow from a fresh gas flow tube connectable thereto; a patient interface end, which is connectable with a patient interface; an outlet comprising an open end; a CPAP generator comprising first, second, and third connection portions, wherein the first connection portion is connected with the fresh gas flow inlet, the second connection portion is connected with the patient interface end, and the third connection portion is connected with the outlet, wherein the CPAP generator is arranged to provide a primary fresh gas flow when receiving the fresh gas flow from the fresh gas flow inlet, and a generated CPAP level is adjustable by varying the fresh gas flow; a secondary flow channel comprising first and second ends, the first end being connected with at least one of the second connection portion, the third connection portion, and the patient interface end, wherein the secondary flow channel is configured to provide a secondary fresh gas flow to be added, at least in the PPV mode, to the primary fresh gas flow; and a flow resistor arranged between the outlet and the first end of the secondary flow channel to provide a resistance to a flow in a direction towards the outlet.

The device may be advantageous in that providing the flow resistor between the outlet and the first end of the secondary flow channel allows manipulation of system inspiratory and expiratory time constants during PPV treatment, and for adjustment of the imposed work of breathing during CPAP treatment. Using adjustments of expiratory time constants and imposed work of breathing to optimize respiratory support of newborn infants has not been described in animals or humans previously. The presented invention thus provides a tool for research in this field and enables optimization of resuscitation and respiratory support, leading to improved respiratory care for infants.

The device can be used not only for infants during the transition from liquid to aerated lungs (establishing functional residual capacity (FRC)) during birth, but also for infants with lung disease, immature lungs, or other conditions. The patients or lungs that would most benefit from optimization of time constants and work of breathing has not been established. It is also possible that this changes over time and it is likely that some effects are beneficial, while others are harmful. The possible benefits or harms of providing an increased or decreased resistance to breathing may change over time and depends on factors such as gestational age, previous mechanical ventilation, use of surfactant, heterogenicity of lung disease, spontaneous breathing effort, grunting, and compliance of the lungs.

The build of FRC is in healthy infants partly accomplished by glottic closure leading to increased expiratory time constants. In small animals, high respiratory rate in combination with the expiratory time constants both contribute to aeration. These normal behaviours indicate that expiratory time constants are an important part of normal respiratory mechanics and physiology.

Further, the device is easy to use both for PPV and CPAP treatments. The device comprising the flow resistor arranged to provide a resistance to a flow in the direction towards the outlet allows rapid and easy adjustment of expiratory time constants and imposed work of breathing, which is advantageous. When used in combination with a pressure stable system, such as that disclosed in WO2012108826, it further allows adjustment and optimization over a very wide range of fresh gas flows, allowing adjustment of inspiratory time and peak inspiratory flows.

For purposes of this invention, the words "infant" and "child" are intended to encompass a patient such as a newborn, and a neonatal child which is in need of neonatal resuscitation and initial respiratory support. The words are also intended to encompass children and toddlers of up to around 10 kg of weight. The fresh gas flow is adapted to the weight and size of the child such that a desirable rise time is attained.

For purposes of this invention, the wording "fresh gas flow" is air, oxygen or a mixture of these that flows through the system and its parts, and the wording "fresh gas flow tube" is wherein the fresh gas flows. The fresh gas flow can be warm and humidified.

For purposes of this invention, the wording "variable flow CPAP generator" is a device intended to encompass any continuous positive airway pressure device where the CPAP level is adjusted by varying the fresh gas flow.

For purposes of this invention, the wording "imposed work of breathing" is the additional work required to breathe through a device and is an established way of measuring the pressure stability of a system. Imposed work of breathing is obtained by calculating the area within a pressure-volume loop for one breath.

For purposes of this invention, the wording "expiratory time constants" is a dynamic measurement of respiratory mechanics and provides information about the actual time needed for complete expiration. The expiratory time constant can be determined by analyses of exhaled tidal volume and flow rate values.

For purposes of this invention, the word "patient interface" is intended to encompass any interface that is suitable for connecting to a patient, e.g. an infant or child, such as a pair of nasal prongs, a mask, an endotracheal tube, or any other suitable device.

There are different ways of arranging the secondary flow channel and the flow resistor in order to achieve the advantages set forth above.

In accordance with an embodiment of the device, the flow resistor is a fixed flow resistor. Thus, according to this embodiment, the flow resistor is arranged to provide a resistance to a flow in the direction towards the outlet, at least, which is fixed to a predetermined level. This is advantageous in that it provides a constant resistance to the flow through the device in the direction towards the outlet.

In accordance with an embodiment of the device, the flow reistor is an adjustable flow resistor. That is, the flow resistance provided by the flow resistor is adjustable. This is advantageous in that the flow resistance provided by the flow resistor can be adjusted and adapted to the patient subject to treatment. The adjustment can for example be made stepwise, gradually, or servo-controlled.

In accordance with an embodiment of the device, the adjustable flow resistor is one of a manually controllable flow resistor, an electrically controllable flow resistor, and a pneumatically controllable flow resistor.

In accordance with an embodiment of the device, the flow resistor is a mechanical flow resistor.

In accordance with an embodiment of the device, the flow resistor comprises an internal diameter defining a flow resistor channel which is in fluid connection with the CPAP generator, and wherein the internal diameter of the flow resistor is smaller than the internal diameter of an adjacent portion of the device with which the flow resistor channel is in fluid connection. In accordance with an embodiment of the device, the internal diameter of the flow resistor channel is smaller than an inner diameter of an adjacent portion of the device which is upstream of the flow resistor with respect to the flow in the direction towards the outlet. Thereby, a resistance to the flow through the device in the direction towards the outlet is provided. The flow resistor may for example be a connector comprising the flow resistor channel. Providing a flow resistor which is a tube comprising the flow resistor channel, or e.g. a filter is, however, also possible within the concept of the present invention.

In accordance with an embodiment of the device, the flow resistor is a flow resistor which is mechanically, electrically, or pneumatically controlled. According to an example, the flow resistor comprises a valve. Valves are commonly available components in aneastesia machines and respirators, but have not previously been used in variable flow CPAP devices for PPV and CPAP treatment to provide a resistance to a flow in a direction towards the outlet and, thereby, adjust and optimize expiratory time constants and imposed work of breathing.

In accordance with an embodiment of the device, the flow resistor is arranged at the outlet.This allows easy access to the resistor and facilitates manual control and replacement of the resistor. In some embodiments, the flow resistor is removably arranged at the outlet. In such embodiments, flow resistors providing different levels of flow resistance may be provided and interchanged depending on the needs for a treatment. Such a flow resistor can, according to some embodiments, comprise a connector comprising the flow resistor channel. By providing several connectors, each with a flow resistor channel of different diameter, which are removably arrangeable at the outlet of the device, and wherein the diameters of the flow resistor channels are smaller than the inner diameter of the third connection portion of the CPAP generator, the flow resistance provided by the flow resistor can be adjusted as desired by interchanging the connector arranged at the outlet. Such connectors may be arranged at the outlet of the device for example by press fitting or threading. Other means for arranging the connector at the outlet are, however, also conceivable within the concept of the present invention.

In accordance with an embodiment of the device, the first end of the secondary flow channel is connected with the patient interface end and the flow resistor is arranged between the patient interface end and the outlet. The flow resistor can in this embodiment be arranged at the second or third connection portion of the CPAP generator, or at the outlet.

In accordance with an embodiment of the device, the first end of the secondary flow channel is connected with the third connection portion and the second end of the secondary flow channel is connected with the patient interface end, and the flow resistor is arranged between the third connection portion and the outlet. Arranging the flow resistor between the third connection portion and the patient interface end is, in this embodiment, not desireable since the flow resistor thus would interfere with the secondary flow channel, leading to an increase in inspiratory time constants, which is disadvantageous in the resuscitation and respiratory treatment.

In accordance with an embodiment of the device, the secondary flow channel comprises a second variable flow CPAP generator arranged to receive a second fresh gas flow. Providing several, e.g. third and fourth variable flow CPAP generators, providing further secondary flow channels, is also possible within the concept of the present invention. Here, the total fresh gas flow and the CPAP level generated when a fresh gas flow is provided to the device can be controlled by engaging or disengaging variable flow CPAP generators, or by directing flow to CPAP generators that are less efficient. In accordance with an embodiment, each variable flow CPAP generator is connected with the fresh gas flow inlet, the patient interface end, and the outlet of the device, providing a compact device.

In accordance with an embodiment of the device, the secondary flow channel is an internal leakage channel, the first end of which is connected with at least one of the patient interface end and the third connection portion and the second end of which is connected with the fresh gas flow inlet, and wherein the internal leakage channel bypasses the first connection portion of the CPAP generator. This provides a compact device.

In accordance with an embodiment of the device, the secondary flow channel comprises a secondary fresh gas flow tube arranged to receive a second fresh gas flow from a fresh gas source provided externally of the device.

According to a second aspect of the invention, there is presented a system for PPV and CPAP treatment comprising a device according to any one of the preceding claims, a fresh gas flow tube, a fresh gas source connected with the fresh gas flow inlet by means of the fresh gas flow tube, and a pressure release valve arranged to prevent an excessive positive pressure in a PPV mode. The system thus includes the fresh gas source and a pressure release valve, which is set to a specific opening pressure adapted to suit the patient to be treated. In accordance with an embodiment of the system, it is arranged such that when the open end of the outlet of the device is occluded, the pressure will increase from the variable flow CPAP generator until an opening pressure of the pressure release valve is reached, which increase in pressure results in an inspiratory flow, whereby the pressure in the system will remain at the set PPV pressure until the occlusion is removed from the outlet. When the outlet is not occluded, the pressure will return to the set CPAP level, whereby the reduction in pressure leads to an expiratory flow. The resusciation system expiratory time constant is related to the expiratory resistance. An increased resistance will increase the time constants. The total expiratory time constant of a patient will also depend on factors other than the resuscitation system, such as patient endotracheal tube size and lung compliance.

In accordance with an embodiment of the system, it is arranged such that during spontaneous breathing, the patient flow and the fresh gas flow leaves the system through the variable flow CPAP generator, keeping the positive pressure within the airway of the patient stable by varying the flow that generated the CPAP, whereby the CPAP in the airway can be adjusted as needed. The resuscitation system imposed work of breathing is related to the expiratory resistance. An increased resistance will increase the imposed work of breathing. The total work of breathing of a patient is the physiologic work (elastic and resistive) plus the imposed work (resistance of the breathing apparatus or resusciation device).

In accordance with an embodiment of the system, the pressure release valve is connected with one of the patient interface end and the outlet of the device. These are two alternative ways of arranging the pressure release valve in the system, where the main aim is to arrange it in a position where the pressure of interest is measurable in a reliable way. Arranging the pressure release valve in connection with the outlet of the device provides a compact device and system.

In accordance with an embodiment of the system, the system further comprises a pressure measuring device. Thereby, it is possible to easily monitor the operation of the system and make desirable adjustments of the fresh gas flow. Preferably, the presure measuring device is connected close to the patient interface end of the device. Naturally, the main aim is to arrange the pressure measuring device in a position where the pressure of interest is measurable in a reliable and accurate way.

The device as disclosed herein can be used in PPV and CPAP treatment for neonatal resuscitation and respiratory support.

The system as disclosed herein can be used in PPV and CPAP treatment for neonatal resuscitation and respiratory support.

According to a third aspect of the disclosure, there is (but not separately claimed) presented a method of adjusting expiratory time constants and imposed work of breathing for PPV and CPAP treatment, the method comprising the steps of providing a system as disclosed herein; and, during use of the system, operating the flow resistor of the system to provide different levels of resistance to a flow in the direction towards the outlet, thereby adjusting expiratory time constant and imposed work of breathing.

Effects and features of the second and third aspects are largely analogous to those described above in connection with the first aspect. Embodiments mentioned in relation to the first aspect are largely compatible with the second and third aspects. It is further noted that the inventive concepts relate to all possible combinations of features unless explicitly stated otherwise.

A further scope of applicability of the present disclosure will become apparent from the detailed description given below. However, it should be understood that the detailed description and specific examples, while indicating preferred embodiments of the disclosure, are given by way of illustration only, since various changes and modifications within the scope of the disclosure will become apparent to those skilled in the art from this detailed description.

Hence, it is to be understood that this disclosure is not limited to the particular component parts of the device described or steps of the methods described as such device and method may vary. It is also to be understood that the terminology used herein is for purpose of describing particular embodiments only and is not intended to be limiting. It must be noted that, as used in the specification and the appended claim, the articles "a", "an", "the", and "said" are intended to mean that there are one or more of the elements unless the context clearly dictates otherwise. Thus, for example, reference to "a unit" or "the unit" may include several devices, and the like. Furthermore, the words "comprising", "including", "containing" and similar wordings does not exclude other elements or steps.

### BRIEF DESCRIPTION OF THE DRAWINGS

The invention will by way of example now be described in more detail and with reference to the appended schematic drawings, in which:
Fig. 1 shows a schematic cross-sectional view of a device and system according to an embodiment of the present invention;
Fig. 2 shows a schematic cross-sectional view of a device and system according to an embodiment of the present invention;
Fig. 3 shows a schematic cross-sectional view of a device and system according to an embodiment of the present invention;
Fig. 4 shows a schematic cross-sectional view of a device and system according to an embodiment of the present invention;
Fig. 5 shows a schematic cross-sectional view of a device according to an embodiment of the present invention;
Fig. 6 shows a schematic, partly cross-sectional view of a device and system according to an embodiment of the present invention;
Fig. 7 shows a graph of expiratory time constants during positive pressure ventialtion;
Fig. 8 shows a graph of imposed work of breathing as a function of CPAP level during spontaneous breathing; and
Fig. 9 shows a graph of imposed work of breathing as a function of CPAP level during spontaneous breathing.

### DESCRIPTION OF EMBODIMENTS

The present disclosure will now be described more fully hereinafter with reference to the accompanying drawings, in which currently preferred embodiments of the disclosure are shown. This disclosure may, however, be embodied in many different forms and should not be construed as limited to the embodiments set forth herein; rather, these embodiments are provided for thoroughness and completeness, and to fully convey the scope of the disclosure to the skilled person.

Common for the embodiments of the device for PPV and CPAP treatment described herein is that they comprise a fresh gas flow inlet 2; a patient interface end 3; an outlet 4 having an open end 5; a variable CPAP generator 6 connected with the fresh gas flow inlet 2, the patient interface end 3, and the outlet 4; a secondary flow channel 7 comprising first and second ends 8, 9; and a flow resistor 10 arranged between the outlet 4 and the first end 8 of the secondary flow channel 7 to provide a resistance to a flow in a direction towards the outlet 4. The arrangement of the secondary flow channel 7 and the flow resistor 10 of the device may vary within the inventive concept, as is illustrated by the different exemplifying embodiments described hereinafter, at least.

Different embodiments of a system comprising the device for PPV and CPAP treatment are also disclosed. Common for the embodiments of the system for PPV and CPAP treatment described herein is that they comprise a device for PPV and CPAP treatment as described herein, a fresh gas flow tube 14 connected with the fresh gas flow inlet 2 of the device, a fresh gas source 20 connected with the fresh gas flow inlet 2 through the fresh gas flow tube 14, and a pressure release valve 16 arranged to prevent an excessive positive pressure in the PPV mode. Optionally, the system further comprises a pressure measuring device 17 arranged to measure the pressure provided to the patient.

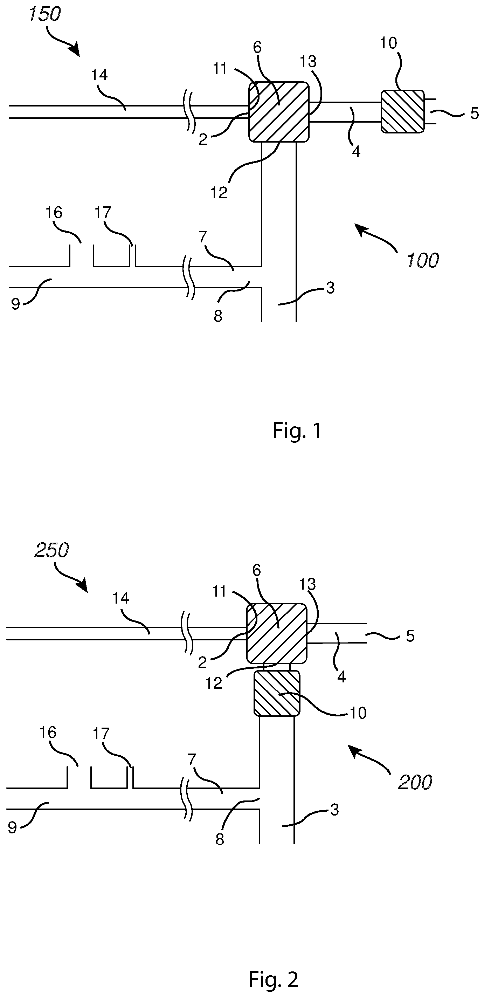

With reference to Fig. 1, according to a first embodiment of the device 100 and system 150 for PPV and CPAP treatment, the device 100 comprises a CPAP generator 6 which is a variable flow CPAP generator comprising a first connection portion 11 connected with a fresh gas flow inlet 2, a second connection portion 12 connected with a patient interface end 3, and a third connection portion 13 connected with an outlet 4 of the device 100. The device 100 further comprises a secondary flow channel 7 comprising first and second ends 8, 9. The first end 8 of the secondary flow channel 7 is in this embodiment connected with the patient interface end 3 of the device 100. The second end 9 of the secondary flow channel 7 is connectable to a fresh gas source for providing a fresh gas flow to the secondary flow channel 7. The device 100 shown in Fig. 1 further comprises a flow resistor 10. The flow resistor 10 is arranged at the outlet 4 and has an open end 5, which constitutes the open end 5 of the device 100. The flow resistor 10 can here be for example one of an electrically controllable adjustable valve, or a connector comprising an internal channel having a diameter which is smaller than that of the adjacent portion of the device 100 with which it is in fluid connection, here the third connection portion 13 and the outlet 4 of the device 100. The connector can be removably arranged at the outlet 4 of the device, for example by press fitting the connector to the open end 5 of the outlet 4. By providing several connectors, each having an internal channel of different diameter with respect to the others, and being removably arrangeable at the outlet 4, e.g. the open end 5 of the device 100, the level of flow resistance provided by the flow resistor 10 can be adjusted by exchanging one connector with another. Such a flow resistor 10 is thus an adjustable flow resistor. As understood from this disclosure, other types of flow resistors are also conceivable within the concept of the present invention, provided that a resistance to a flow in the direction towards the open end 5 of the device is provided. For example, a flow resistor may be provided which has an opening that is manually adjustable in size. The flow resistor may further be a valve which is manually, electrically or pneumatically adjustable.

The device 100 is here integrated in a system 150 for PPV and CPAP treatment. The system further comprises a fresh gas flow tube 14 which is connected to the fresh gas flow inlet 2 of the device 100. The fresh gas flow tube 14 is, at the end opposite to that connected with the fresh gas flow inlet 2, connectable to a fresh gas source for providing a fresh gas flow to the device 100. Further, in this embodiment of the system 150, a pressure release valve 16 is provided in fluid connection with the secondary flow channel 7. A pressure measuring device 17 is also arranged in connection with the secondary flow channel 7 between the first end 8 and the pressure release valve 16. The pressure measuring device 17 may, however, be omitted in all embodiments if there is no interest in measuring the pressure.

With reference to Fig. 2, according to a second embodiment of the device 200 and system 250, resembling the device 100 and system 150 described with reference to Fig. 1 apart from the arrangement of the flow resistor 10, the flow resistor 10 is arranged between the patient interface end 3 and the second connection portion 12 of the CPAP generator 6. The first end 8 of the secondary flow channel 7 is also here connected with the patient interface end 3 of the device 200. The flow resistor 10 is thus arranged between the first end 8 of the secondary flow channel 7 and the outlet 4 of the device 200 to provide a resistance to a flow in the direction towards the outlet 4.

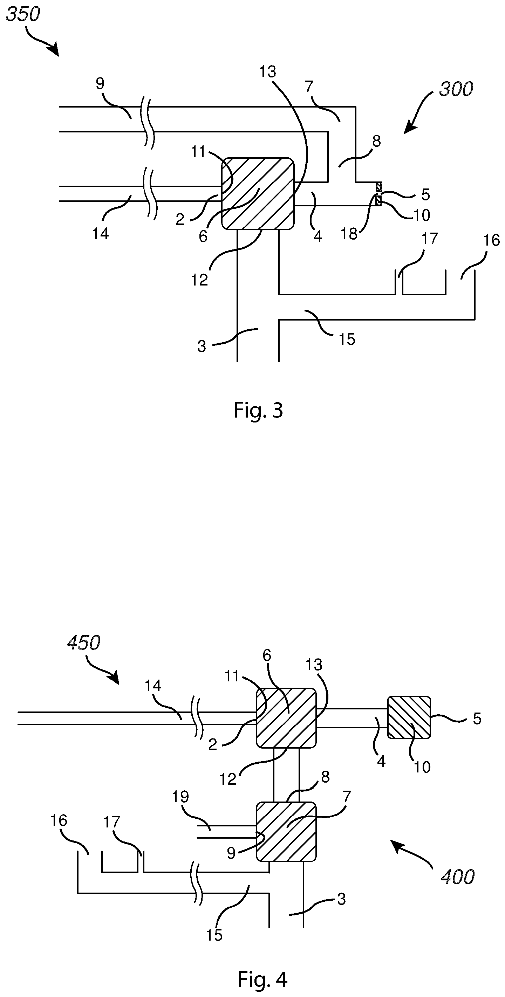

A third embodiment of the device 300 and system 350 for PPV and CPAP treatment is shown in Fig. 3. In this exemplifying embodiment, the secondary flow channel 7 is arranged in connection with the third connection portion 13 of the CPAP generator 6 of the device 300. The second end 9 of the secondary flow channel 7 is, as for the embodiments shown in Figs. 1-2, connectable to a fresh gas source for providing a fresh gas flow to the secondary flow channel 7. In this embodiment, the flow resistor is also arranged at the outlet 4 of the device 300 and downstream of the first end 8 of the secondary flow channel 7, considering a direction of flow from the patient interface end 3 to the open end 5 of the outlet 4 of the device 300. The flow resistor 10 is thus arranged between the first end 8 of the secondary flow channel 7 and the open end 5 of the outlet 4 of the device 300. More particularly, in this embodiment, the flow resistor 10 comprises an internal diameter defining a flow resistor channel 18 which is in fluid connection with the CPAP generator 6 through the third connection portion 13 and the outlet 4. As can be seen in Fig. 3, the internal diameter of the flow resistor channel 18 is smaller than the internal diameter of the adjacent portion of the outlet 4 with which the flow resistor 10 is in fluid connection. More particularly, the internal diameter of the flow resistor channel 18 is smaller than the internal diameter of the adjacent upstream portion of the device 300 with which the flow resistor 10 is in fluid connection. The downstream end of the flow resistor channel 18 here constitutes the open end 5 of the device 300.

In the exemplifying embodiment shown in Fig. 3, the device 300 is further integrated in a system 350 comprising a pressure release tube 15, a pressure release valve 16, and a pressure measuring device 17. Here, the pressure release tube 15 is connected, at a first end, with the patient interface end 3. The pressure release valve 16 is arranged at a second end of the pressure release tube 15, opposite the first end of the pressure release tube 15. The pressure measuring device 17 is arranged between the patient interface end 3 and the pressure release valve 16.

A fourth embodiment of the device 400 and system 450 for PPV and CPAP treatment is shown in Fig. 4. In this embodiment of the device 400, the flow resistor is arranged at the outlet 4 as in the embodiments described with respect to Figs. 1 and 3. Further, the secondary flow channel 7 is arranged in connection with the patient interface end 3 and the second connection portion 12 of the CPAP generator 6. The secondary flow channel 7 here comprises a second CPAP generator arranged to receive a second fresh gas flow from a second fresh gas flow tube 19 connected thereto. The second CPAP generator is thus here connected to the second fresh gas flow tube 19, the patient interface end 3 and, through the CPAP generator 6 and more particularly the second connection portion 12 thereof, with the outlet 5 of the device 400. More particularly, the secondary flow channel 7 comprises a first end 8 corresponding to a third connection portion of the second CPAP generator, which is connected with the second connection portion 12 of the CPAP generator 6, and a second end 9, corresponding to a first connection portion of the second CPAP generator, which is connectable with the second fresh gas flow tube 19. The third connection portion of the second CPAP generator is connected with the patient interface end 3. The device 400 is comprised in a system 450 further comprising a pressure release tube 15 comprising a pressure release valve 16 and a pressure measuring device 17 as described with respect to the embodiment shown in Fig. 3.

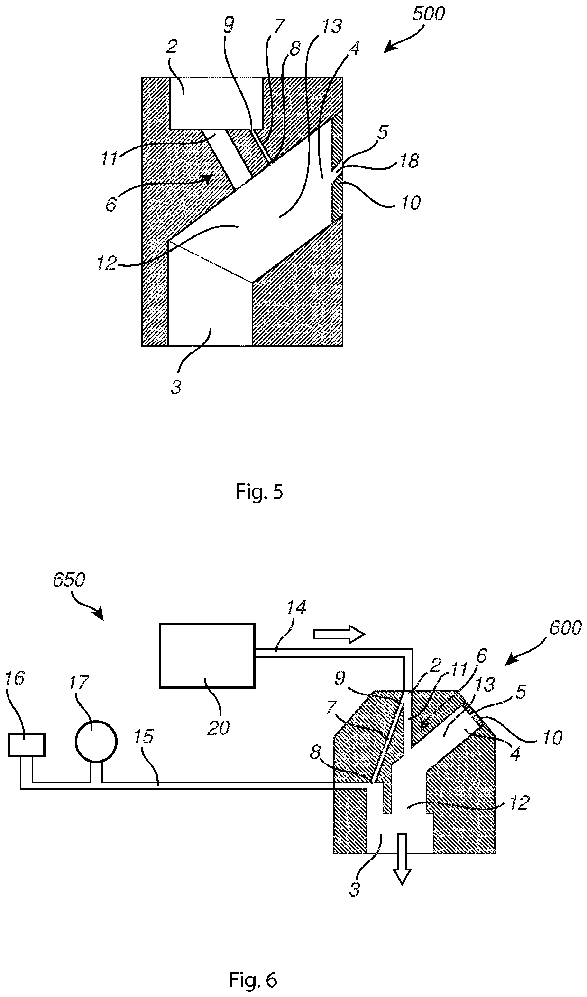

Yet another embodiment of the device 500 for PPV and CPAP treatment is shown in Fig. 5. In this embodiment, the flow resistor 10 is arranged at the outlet 4 of the device 500 as described also with respect to Figs. 1, 3, and 4. The secondary flow channel 7 of this embodiment is an internal leakage channel which extends between the fresh gas flow inlet 2 and the third connection portion 13 of the CPAP generator 6, bypassing the first connection portion 11 of the CPAP generator 6. That is, the first end 8 of the secondary flow channel 7 is connected with the third connection portion 13 of the CPAP generator 6 and and the second end 9 of the secondary flow channel 7 is connected with the fresh gas flow inlet 2 of the CPAP generator 6 of the device 500.

Fig. 6 shows yet another embodiment of the device 600 and a system 650 for PPV and CPAP treatment. The flow resistor 10 of the device 600 is arranged at the outlet 4 as in the embodiments described with respect to Figs. 1 and 3-5. In this embodiment, however, the secondary flow channel 7 is an internal leakage channel comprising a first end 8 which is connected with the patient interface end 3, and a second end 9 which is connected with the fresh gas flow inlet 2. The internal leakage channel 7 thus bypasses the first connection portion 11 of the CPAP generator 6. In this exemplifying embodiment, the system 650 comprises the device 600 for PPV and CPAP treatment and a fresh gas flow tube 14, which is connected to the fresh gas flow inlet 2 of the device 600. The system 650 further comprises a fresh gas source 20 to which the fresh gas flow tube 14 is connected. Further, the system 650 comprises a pressure release tube 15 connected to the patient interface end 3 of the device 600. The pressure release tube 15 is connected with a pressure release valve 16 and a pressure measuring device 17, and the pressure measuring device 17 is arranged between the patient interface end 3 and the pressure release valve 16.

The system is operated as follows for PPV and CPAP treatment. Reference will be made to the first embodiment of the system 150, but the other embodiments disclosed herein have a corresponding operation. Oxygen concentration and fresh gas flow are adjusted by a standard blender and a flow meter. The fresh gas flow can be varied and is typically set to between 5 and 15 litres per minute. Typically, the fresh gas flow provided is between 10 and 12 litres per minute. A fresh gas flow in this range should prevent rebreathing, provide flow to achieve an adequate inspiration flow, volume and time, and provide some allowance for leakage at the patient interface. However, a fresh gas flow above 15 litres per minute is also possible to provide, particularly for treatment of an older child.

The fresh gas flow is used to drive the CPAP generator 6 and is adjustable, thus making the CPAP generator 6 a variable flow CPAP generator 6. The system is further configured such that this range of supplied fresh gas flow generates a level of CPAP of 3-10 cm H2O.

During treatment, if the open end 5 of the outlet 4 of the device 100 is occluded, the pressure delivered to the patient will increase from the pressure set by the variable CPAP generator 6 unitl the opening pressure of the pressure release valve 16 is reached. A typical value for the pressure release valve to open is around 20-30 cm H2O. The increase in pressure results in an inspiratory flow. The pressure in the system 100 will remain at the set positive pressure ventilation pressure until the outlet occlusion is removed. When the occlusion is removed from the open end 5, the pressure delivered to the patient will return to the set CPAP level and the resulting reduction in pressure will lead to an expiratory flow.

During spontaneous breathing, the infant flow and the fresh gas flow leaves the system 100 through the variable flow CPAP generator 6. This keeps the positive pressure within the airway stable. By varying the flow, i.e. the fresh gas flow, that generated the CPAP pressure, the CPAP pressure in the airway can be adjusted as needed.

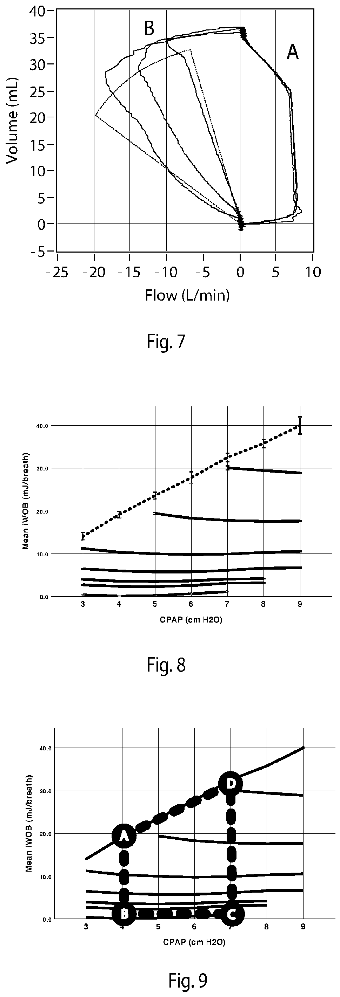

The use of a device comprising a flow resistor as herein disclosed allows adjusting the expiratory time constants during positive pressure ventilation treatment. The effect of being able to adjust expiratory time constants is evident from the graph of Fig. 7, where the loops represent three recordings with identical inspiration (A) and with exhalations (B) for which the expiratory time constants have been adjusted (dashed line wedge). With reductions in expiratory time constants, the expiratory flows increase (as observed from the X-axis negative maximum). The example was recorded at a fresh gas flow of 10 L/min with positive pressure ventilation in a simulated lung model of an infant.

The use of a device comprising a flow resistor as herein disclosed also allows adjusting the imposed work of breathing during CPAP treatment during which the patient breaths spontaneously. The effect of being able to adjust the imposed work of breathing is evident from Figure 8, showing a known T-piece system in dashed line, which represents the maximum possible imposed work of breathing at a given level of CPAP. The solid lines show how maintaning a selected level of imposed work of breathing for different levels of CPAP is possible with the device and system comprising a flow resistor as herein disclosed. Further, the imposed work of breathing can be reduced to close to zero in a controlled and predicatable way as decided by the user. The example was recorded at a fresh gas flow of 10 L/min with simulated breathing in a lung model of an infant.

Figure 9 shows an example of an experiment or optimization. The disclosed invention allows the user to adjust CPAP and imposed work of breathing (iWOB) from low to high in a controlled manner. At (A) the CPAP is low and iWOB is high, at (B) the CPAP and iWOB are both low, at (C) the CPAP is increased with low iWOB, and at (D) the CPAP is increased and the iWOB is high. The device and system herein disclosed thus allow easy adjustment within the dashed area and control of both iWOB and CPAP.

The patient interface end 3 can be designed in any suitable form such to be suitable for connection with a patient interface. Further, the patient interface can assume a variety of designs suitable for establishing a connection to the patient nasal airways. Thus, the pateint interface can include an opposing pair of nasal prongs, a mask, an endotracheal tube, or any other suitable devices.

The system can have a backup system for malfunctioning of the pressure release valve 16. This could either be an alarm, a second release valve, or a system that cuts the fresh gas flow.

The pressure measuring device 17 should be positioned as close to the patient as possible to provide accurate recording of the pressure of the gas delivered to the patient. The accuracy will depend on the flow resistance of the patient interface and, for an infant, a low resistance interface should be used if possible.

Notably, the device, in accordance with principles of the present invention is useful with a wide variety of patient interface configurations that may or may not incorporate some or all of the features described above with respect to the patient interface. Thus, the patient interface is in no way limiting.

Notably, the device, in accordance with principles of the present invention is useful with a wide variety of variable flow CPAP generators that may or may not incorporate some or all of the features described above with respect to the variable flow CPAP generator 6. Thus, the model of the variable flow CPAP generator 6 is in no way limiting.

Notably, the system, in accordance with principles of the present invention is useful with a wide variety of pressure release valves 16 or similar devices that achieve the purpose of releasing air depending on the pressure in the system. Thus, the model or type of pressure release valve is in no way limiting.

Notably, the system, in accordance with principles of the present invention is useful with a wide variety of pressure measuring devices 17 or similar devices that achieves the purpose of measuring the pressure in the system. Thus, the model or type pressure measuring devices is in no way limiting.

A typical CPAP level for resuscitating or stabilising an infant is in the range of 3-10 cm H2O. A typical peak pressure for PPV is 20-30 cm H2O.

While the invention has been illustrated and described in detail in the drawings and foregoing description, such illustration and description are to be considered illustrative or exemplary and not restrictive. The invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A device for positive pressure ventilation (PPV) and continuous positive airway pressure (CPAP) treatment comprising:
- a fresh gas flow inlet (2), arranged to receive a fresh gas flow from a fresh gas flow tube (14) connectable thereto;
- a patient interface end (3), which is connectable with a patient interface;
- an outlet (4) comprising an open end (5);
- a CPAP generator (6) comprising first, second, and third connection portions, wherein the first connection portion (11) is connected with the fresh gas flow inlet, the second connection portion (12) is connected with the patient interface end and the third connection portion connected with the outlet, wherein the CPAP generator is arranged to provide a primary fresh gas flow when receiving the fresh gas flow from the fresh gas flow inlet, and a generated CPAP level is adjustable by varying the fresh gas flow;
- a secondary flow channel (7) comprising first and second ends, the first end being connected with at least one of the second connection portion, the third connection portion, and the patient interface end, wherein the secondary flow channel is configured to provide a secondary fresh gas flow to be added, at least in the PPV mode, to the primary fresh gas flow; and **characterised in that**
- a flow resistor (10) is arranged between the outlet and the first end of the secondary flow channel to provide a resistance to a flow in a direction towards the outlet.

2. The device for PPV and CPAP treatment according to claim 1, wherein the flow resistor is arranged at the outlet.

3. The device for PPV and CPAP treatment according to claim 1, wherein the first end of the secondary flow channel is connected with the patient interface end and the flow resistor is arranged between the patient interface end and the outlet.

4. The device for PPV and CPAP treatment according to claim 1, wherein the first end of the secondary flow channel is connected with the third connection portion and the second end of the secondary flow channel is connected with the patient interface end, and the flow resistor is arranged between the third connection portion and the outlet.

5. The device for PPV and CPAP treatment according to any one of the preceding claims, wherein the secondary flow channel comprises a second CPAP generator arranged to receive a second fresh gas flow.

6. The device for PPV and CPAP treatment according to any one of claims 1-4, wherein the secondary flow channel is an internal leakage channel, the first end of which is connected with at least one of the patient interface end and the third connection portion and the second end of which is connected with the fresh gas flow inlet, and wherein the internal leakage channel bypasses the first connection portion of the CPAP generator.

7. The device for PPV and CPAP treatment according to any one of claims 1-4, wherein the secondary flow channel comprises a secondary fresh gas flow tube arranged to receive a second fresh gas flow from a fresh gas source provided externally of the device.

8. The device for PPV and CPAP treatment according to any one of the preceding claims, wherein the flow resistor is a fixed flow resistor.

9. The device for PPV and CPAP treatment according to any one of claims 1-7, wherein the flow resistor is an adjustable flow resistor.

10. The device for PPV and CPAP treatment according to claim 9, wherein the adjustable flow resistor is one of a manually controllable flow resistor, an electrically controllable flow resistor, and a pneumatically controllable flow resistor.

11. The device for PPV and CPAP treatment according to any one of the preceding claims, wherein the flow resistor is a mechanical flow resistor.

12. The device for PPV and CPAP treatment according to claim 11, wherein the flow resistor comprises an internal diameter defining a flow resistor channel which is in fluid connection with the CPAP generator, and wherein the internal diameter of the flow resistor channel is smaller than an internal diameter of an adjacent portion of the device with which the flow resistor channel is in fluid connection.

13. The device for PPV and CPAP treatment according to claim 12, wherein the internal diameter of the flow resistor channel is smaller than an inner diameter of an adjacent portion of the device which is upstream of the connector with respect to the flow in the direction towards the outlet.

14. A system for PPV and CPAP treatment comprising a device according to any one of the preceding claims, a fresh gas flow tube, a fresh gas source connected with the fresh gas flow inlet by means of the fresh gas flow tube, and a pressure release valve arranged to prevent an excessive positive pressure in a PPV mode.

15. The system according to claim 14, wherein the pressure release valve is connected with one of the patient interface end and the outlet of the device.

16. The system according to claim 14 or 15, further comprising a pressure measuring device.

## Patentansprüche

1. Vorrichtung zur Behandlung mit Positivdruckbeatmung (PPV für *Positive Pressure Ventilation)* und kontinuierlichem positivem Atemwegsdruck (CPAP für *Continuous Positive Airway Pressure),* umfassend:
- einen Frischgasströmungseinlass (2), der angeordnet ist, um eine Frischgasströmung von einer Frischgasströmungsleitung (14) aufzunehmen, die damit verbindbar ist;
- ein Patientenschnittstellenende (3), das mit einer Patientenschnittstelle verbindbar ist;
- einen Auslass (4), der ein offenes Ende (5) umfasst;
- einen CPAP-Generator (6), der einen ersten, zweiten und dritten Verbindungsabschnitt umfasst, wobei der erste Verbindungsabschnitt (11) mit dem Frischgasströmungseinlass verbunden ist, der zweite Verbindungsabschnitt (12) mit dem Patientenschnittstellenende verbunden ist und der dritte Verbindungsabschnitt (13) mit dem Auslass verbunden ist, wobei der CPAP-Generator angeordnet ist, um eine Primärfrischgasströmung vorzusehen, wenn er die Frischgasströmung von dem Frischgasströmungseinlass empfängt, und ein erzeugter CPAP-Pegel durch Variieren der Frischgasströmung einstellbar ist;
- einen Sekundärströmungskanal (7), der ein erstes und ein zweites Ende umfasst, wobei das erste Ende mit dem zweiten Verbindungsabschnitt, dem dritten Verbindungsabschnitt und/oder dem Patientenschnittstellenende verbunden ist, wobei der Sekundärströmungskanal eingerichtet ist, um mindestens in dem PPV-Modus eine Sekundärfrischgasströmung vorzusehen, die der Primärfrischgasströmung hinzugefügt wird; und
**dadurch gekennzeichnet, dass**
- ein Strömungswiderstand (10) zwischen dem Auslass und dem ersten Ende des Sekundärströmungskanals angeordnet ist, um einen Widerstand gegen eine Strömung in Richtung zu dem Auslass vorzusehen.

2. Vorrichtung zur PPV- und CPAP-Behandlung nach Anspruch 1, wobei der Strömungswiderstand an dem Auslass angeordnet ist.

3. Vorrichtung zur PPV- und CPAP-Behandlung nach Anspruch 1, wobei das erste Ende des Sekundärströmungskanals mit dem Patientenschnittstellenende verbunden ist und der Strömungswiderstand zwischen dem Patientenschnittstellenende und dem Auslass angeordnet ist.

4. Vorrichtung zur PPV- und CPAP-Behandlung nach Anspruch 1, wobei das erste Ende des Sekundärströmungskanals mit dem dritten Verbindungsabschnitt verbunden ist und das zweite Ende des Sekundärströmungskanals mit dem Patientenschnittstellenende verbunden ist und der Strömungswiderstand zwischen dem dritten Verbindungsabschnitt und dem Auslass angeordnet ist.

5. Vorrichtung zur PPV- und CPAP-Behandlung nach einem der vorhergehenden Ansprüche, wobei der Sekundärströmungskanal einen zweiten CPAP-Generator umfasst, der angeordnet ist, um eine zweite Frischgasströmung zu empfangen.

6. Vorrichtung zur PPV- und CPAP-Behandlung nach einem der Ansprüche 1-4, wobei der Sekundärströmungskanal ein interner Leckagekanal ist, dessen erstes Ende mit dem Patientenschnittstellenende und/oder dem dritten Verbindungsabschnitt verbunden ist und dessen zweites Ende mit dem Frischgasströmungseinlass verbunden ist, und wobei der interne Leckagekanal den ersten Verbindungsabschnitt des CPAP-Generators umgeht.

7. Vorrichtung zur PPV- und CPAP-Behandlung nach einem der Ansprüche 1-4, wobei der Sekundärströmungskanal eine Sekundärfrischgasströmungsleitung umfasst, die angeordnet ist, um einen zweiten Frischgasstrom von einer Frischgasquelle zu empfangen, die außerhalb der Vorrichtung vorgesehen ist.

8. Vorrichtung zur PPV- und CPAP-Behandlung nach einem der vorhergehenden Ansprüche, wobei der Strömungswiderstand ein fester Strömungswiderstand ist.

9. Vorrichtung zur PPV- und CPAP-Behandlung nach einem der Ansprüche 1-7, wobei der Strömungswiderstand ein einstellbarer Strömungswiderstand ist.

10. Vorrichtung zur PPV- und CPAP-Behandlung nach Anspruch 9, wobei der einstellbare Strömungswiderstand einer von einem manuell steuerbaren Strömungswiderstand, einem elektrisch steuerbaren Strömungswiderstand und einem pneumatisch steuerbaren Strömungswiderstand ist.

11. Vorrichtung zur PPV- und CPAP-Behandlung nach einem der vorhergehenden Ansprüche, wobei der Strömungswiderstand ein mechanischer Strömungswiderstand ist.

12. Vorrichtung zur PPV- und CPAP-Behandlung nach Anspruch 11, wobei der Strömungswiderstand einen Innendurchmesser umfasst, der einen Strömungswiderstandskanal definiert, der in Fluidverbindung mit dem CPAP-Generator steht, und wobei der Innendurchmesser des Strömungswiderstandskanals kleiner ist als ein Innendurchmesser eines benachbarten Abschnitts der Vorrichtung, mit dem der Strömungswiderstandskanal in Fluidverbindung steht.

13. Vorrichtung zur PPV- und CPAP-Behandlung nach Anspruch 12, wobei der Innendurchmesser des Strömungswiderstandskanals kleiner ist als ein Innendurchmesser eines benachbarten Abschnitts der Vorrichtung, der stromaufwärts von dem Verbinder in Bezug auf die Strömung in der Richtung zu dem Auslass liegt.

14. System zur PPV- und CPAP-Behandlung, umfassend eine Vorrichtung nach einem der vorhergehenden Ansprüche, eine Frischgasströmungsleitung, eine Frischgasquelle, die mittels der Frischgasströmungsleitung mit dem Frischgasströmungseinlass verbunden ist, und ein Druckentlastungsventil, das angeordnet ist, um einen übermäßigen Überdruck in einem PPV-Modus zu verhindern.

15. System nach Anspruch 14, wobei das Druckentlastungsventil mit dem Patientenschnittstellenende und dem Auslass der Vorrichtung verbunden ist.

16. System nach Anspruch 14 oder 15, ferner umfassend eine Druckmessvorrichtung.

## Revendications

1. Dispositif de ventilation à pression positive (PPV) et traitement par ventilation à pression positive continue (CPAP) comprenant :
- une entrée de flux de gaz frais (2), conçue pour recevoir un flux de gaz frais à partir d'un tube d'écoulement de gaz frais (14) apte à être raccordé à celle-ci ;
- une extrémité d'interface patient (3), apte à être raccordée à une interface patient ;
- une sortie (4) comprenant une extrémité ouverte (5) ;
- un générateur de CPAP (6) comprenant des première, deuxième et troisième parties de raccordement, dans lesquels la première partie de raccordement (11) est raccordée à l'entrée de flux de gaz frais, la deuxième partie de raccordement (12) est raccordée à l'extrémité d'interface patient et la troisième partie de raccordement (13) est raccordée à la sortie, dans lesquels le générateur de CPAP est conçu pour fournir un flux de gaz frais primaire lors de la réception du flux de gaz frais à partir de l'entrée de flux de gaz frais, et un niveau de CPAP généré peut être réglé en modifiant le flux de gaz frais ;
- un canal d'écoulement secondaire (7) comprenant des première et deuxième extrémités, la première extrémité étant raccordée à l'une au moins parmi la deuxième partie de raccordement, la troisième partie de raccordement et l'extrémité d'interface patient, dans lesquels le canal d'écoulement secondaire est configuré pour fournir un flux de gaz frais secondaire destiné à être ajouté, au moins dans le mode PPV, au flux de gaz frais primaire ; et
**caractérisé en ce que**
- un limiteur d'écoulement (10) est agencé entre la sortie et la première extrémité du canal d'écoulement secondaire pour fournir une résistance à un écoulement dans une direction vers la sortie.

2. Dispositif de PPV et traitement de CPAP selon la revendication 1, dans lesquels le limiteur d'écoulement est agencé au niveau de la sortie.

3. Dispositif de PPV et traitement de CPAP selon la revendication 1, dans lesquels la première extrémité du canal d'écoulement secondaire est raccordée à l'extrémité d'interface patient et le limiteur d'écoulement est agencé entre l'extrémité d'interface patient et la sortie.

4. Dispositif de PPV et traitement de CPAP selon la revendication 1, dans lesquels la première extrémité du canal d'écoulement secondaire est raccordée à la troisième partie de raccordement et la deuxième extrémité du canal d'écoulement secondaire est raccordée à l'extrémité d'interface patient, et le limiteur d'écoulement est agencé entre la troisième partie de raccordement et la sortie.

5. Dispositif de PPV et traitement de CPAP selon l'une quelconque des revendications précédentes, dans lesquels le canal d'écoulement secondaire comprend un deuxième générateur de CPAP conçu pour recevoir un deuxième flux de gaz frais.

6. Dispositif de PPV et traitement de CPAP selon l'une quelconque des revendications 1 à 4, dans lesquels le canal d'écoulement secondaire est un canal de fuite interne, dont la première extrémité est raccordée à l'une au moins parmi l'extrémité d'interface patient et la troisième partie de raccordement et dont la deuxième extrémité est raccordée à l'entrée de flux de gaz frais, et dans lesquels le canal de fuite interne contourne la première partie de raccordement du générateur de CPAP.

7. Dispositif de PPV et traitement de CPAP selon l'une quelconque des revendications 1 à 4, dans lesquels le canal d'écoulement secondaire comprend un tube d'écoulement de gaz frais secondaire conçu pour recevoir un deuxième flux de gaz frais à partir d'une source de gaz frais disposée à l'extérieur du dispositif.

8. Dispositif de PPV et traitement de CPAP selon l'une quelconque des revendications précédentes, dans lesquels le limiteur d'écoulement est un limiteur d'écoulement fixe.

9. Dispositif de PPV et traitement de CPAP selon l'une quelconque des revendications 1 à 7, dans lesquels le limiteur d'écoulement est un limiteur d'écoulement réglable.

10. Dispositif de PPV et traitement de CPAP selon la revendication 9, dans lesquels le limiteur d'écoulement réglable est l'un parmi un limiteur d'écoulement à commande manuelle, un limiteur d'écoulement à commande électrique et un limiteur d'écoulement à commande pneumatique.

11. Dispositif de PPV et traitement de CPAP selon l'une quelconque des revendications précédentes, dans lesquels le limiteur d'écoulement est un limiteur d'écoulement mécanique.

12. Dispositif de PPV et traitement de CPAP selon la revendication 11, dans lesquels le limiteur d'écoulement comprend un diamètre intérieur définissant un canal de limitation d'écoulement, lequel est en communication fluidique avec le générateur de CPAP, et dans lesquels le diamètre intérieur du canal de limitation d'écoulement est inférieur à un diamètre intérieur d'une partie adjacente du dispositif avec lequel le canal de limitation d'écoulement est en communication fluidique.

13. Dispositif de PPV et traitement de CPAP selon la revendication 12, dans lesquels le diamètre intérieur du canal de limitation d'écoulement est inférieur à un diamètre intérieur d'une partie adjacente du dispositif située en amont du raccord par rapport à l'écoulement dans la direction vers la sortie.

14. Système de PPV et traitement de CPAP comprenant un dispositif selon l'une quelconque des revendications précédentes, un tube d'écoulement de gaz frais, une source de gaz frais raccordée à l'entrée de flux de gaz frais au moyen du tube d'écoulement de gaz frais, et une soupape de décharge de pression conçue pour empêcher une pression positive excessive dans un mode PPV.

15. Système selon la revendication 14, dans lequel la soupape de décharge de pression est raccordée à l'une parmi l'extrémité d'interface patient et la sortie du dispositif.

16. Système selon la revendication 14 ou 15, comprenant en outre un dispositif de mesure de pression.
